Europäisches Patentamt

European Patent Office

Office européen des brevets

(11) Publication number : **0 141 607 B2**

# NEW EUROPEAN PATENT SPECIFICATION

(45) Date of publication of the new patent
specification : **03.08.94 Bulletin 94/31**

(51) Int. Cl.[5] : **G02C 13/00, C11D 7/42,
A61L 2/18**

(21) Application number : **84307264.6**

(22) Date of filing : **22.10.84**

(54) Improved method for enxymatic cleaning and disinfecting contact lenses.

(30) Priority : **24.10.83 US 545314**

(43) Date of publication of application :
**15.05.85 Bulletin 85/20**

(45) Publication of the grant of the patent :
**21.12.88 Bulletin 88/51**

(45) Mention of the opposition decision :
**03.08.94 Bulletin 94/31**

(84) Designated Contracting States :
**AT BE CH DE FR GB IT LI LU NL SE**

(56) References cited :
**EP-A- 0 005 131
DE-A- 2 854 278
JP-A-50 064 303
US-A- 3 590 121
US-A- 3 623 956
US-A- 3 632 957
US-A- 3 910 296
US-A- 4 285 738
Hisao Magatani: Acta: XXIV International Congress of Ofthalmology -San Francisco,
October 31- November 5, 1982, vol. 2, pp
1132-1135, published by J.B. Lippincott Co.
R.M. Hill & J. Goings: Contact Lens Forum,
September 1980, pp 54, 55
K. Bhadur and R. Chandra Sinha: Enzymologia, XX, no. 1, 2-20 (1957).
M. Bares et al.: Scientific Papers of Prague
Institute of Chemical Technology, E 45, 97-110
(1976).**

(56) References cited :
**K. Aunstrup:"Economic Microbiology",vol. 5,
"Microbial Enzymes and Bioconversions", pp
49-69, Academic Press, 1980.
A.J. Philips, The Ofthalmic Optician (The Contact Lens Review), 20, no. 11, 375-388 (May 24,
1980).
Bausch & Lomb: Patient Instructions for wearers of Soft Contact Lenses, revised 1/81.
Microbiological Guidelines of FDA, May, 1980
concerning sterilization of contact lenses.
R.A. Moore et al., Contacto, March 1980, 23-30.
T. Bilbaut et al., Exp. Eye Res., 53, 153-165
(1986).
H.W. Hind, Contact Lens Forum, November
1979, 17-27.**

(73) Proprietor : **BAUSCH & LOMB
INCORPORATED
1400 North Goodman Street
Rochester New York 14609 (US)**

(72) Inventor : **Ogunbiyi, Lai
9 Morningview Drive
Fairport New York 14450 (US)**
Inventor : **Riedhammer, Thomas Martin
52 Appian Drive
Rochester New York 14606 (US)**
Inventor : **Smith, Francis Xavier
4281 Downs Road
Walworth New York 14563 (US)**

(74) Representative : **Allam, Peter Clerk et al
LLOYD WISE, TREGEAR & CO.
Norman House
105-109 Strand
London WC2R 0AE (GB)**

EP 0 141 607 B2

## Description

The present invention relates to an improved method for cleaning lenses. More specifically, this invention relates to an improved method for cleaning and disinfecting contact lenses through the use of enzymatic cleaners wherein the time interval needed to complete the process can be significantly reduced thereby providing greater flexibility and convenience to the wearer.

In the normal course of wearing contact lenses, tear films and debris consisting of proteinaceous, oily and sebaceous matter have a tendency to deposit and build-up on lens surfaces. As part of the routine care regimen, hydrophilic gel lenses or soft contact lenses, for example, must be cleaned to remove these tear film deposits and debris. Otherwise, if not removed, both wettability and optical clarity of the lenses can be reduced. Because the composition of such deposits is mainly proteinaceous, enzyme cleaning solutions containing mainly proteolytic enzymes have been widely used.

Although most enzyme cleaners are generally regarded as efficacious and safe, they nevertheless have certain shortcomings which create inconveniences for the user. Heretofore, the process of cleaning contact lenses with enzymatic cleaners involved two main steps: the first constisting of the cleaning phase whereby coated lenses are soaked in the enzyme solution at ambient temperature conditions, i.e.-cold soaking, for a period of at least 2 hours and up to 12 hours, and occasionally, overnight to achieve effective cleaning. At the conclusion of the cleaning step, the lenses must be disinfected. Disinfection normally requires exposing the lenses to a temperature of 80°C for at least 10 minutes. This is carried out by first immersing the lenses in a lens storage case with a second solution, such as preserved isotonic sterile saline followed by asepticizing in a heat disinfection unit. Such units have a heating cycle which normally takes about 1 hour to complete. Thus, the time needed to clean and disinfect soft contact lenses utilizing enzymatic cleaners can take up to 13 hours. Accordingly, there is a need for an abbreviated, more convenient means for cleaning contact lenses with enzyme cleaners which provides a greater degree of flexibility.

In EP-A-0005131 there is disclosed a two-step process for cleaning soft contact lenses in which, in the first step, the lenses are contacted at ambient temperature with a cleaning solution containing a lipolytic enzyme and optionally a proteolytic enzyme, whereafter, in the second step, the lenses are rinsed and boiled in a physiological saline solution. Although it is suggested that the total lens-cleaning time for this process can be relatively short, it nonetheless still involves the use of two separate solutions, nmely the enzymatic cleaning solution and the physiological saline solution.

The present invention provides a less complex and more convenient regimen for enzymatic cleaning of contact lenses whereby actual cleaning and disinfection are carried out in the enzyme solution in a single step over a time period of 60 minutes or less. The method of the present invention provides the added benefit of concurrent deactivation of the active enzymes by the time the cycle is completed. In addition to the greater convenience factor for the user, the improved method for enzymatic cleaning is also more economic since only a single lens care solution is needed; whereas, previous methods required both a cleaning solution and another rinsing solution.

More particularly, the present invention provides a method of (1) cleaning contact lenses of proteinaceous and other deposits using a proteolytic enzyme in aqueous solution, and (2) disinfecting the contact lenses, characterized in that it is a single step method providing simultaneous cleaning and disinfection which comprises placing the lenses in a solution comprising the proteolytic enzyme dissolved in water and then subjecting the solution and lenses to a heating cycle consisting of a heating phase followed by a cooling phase, said heating phase consisting of gradually elevating the temperature of the solution from ambient temperature to a maximum temperature between 60° and 100°C and then maintaining said maximum temperature for not more than 20 minutes, the total time for which the lenses are heated at between 60°C and 100°C being 60 minutes or less, sufficient to clean and disinfect the lenses and inactivate the proteolytic enzyme.

As indicated above, this invention relates to a novel, single step method for cleaning and disinfecting contact lenses, ie one in which a single solution is used to simultaneously clean and disinfect the lenses. The method is adaptable for use with most contact lenses, including hard and soft lenses, as well as the newer hard gas permeable type contact lenses, such as described in U.S. Patent 4,327,203. The invention also may be used with those soft lenses generally referred to as extended-wear lenses containing 55% or more water content. The term "soft contact lens" as used herein generally refers to those lenses which readily flex under small amounts of force and return to their original shape when that force is released. Typically, soft contact lenses are formulated from poly(hydroxyethyl methacrylate) which has been in the preferred formulations cross-linked with ethylene glycol dimethacrylate. For convenience purposes, this polymer is generally known as PHEMA. Soft contact lenses are also made from silicon polymer cross-linked, for example, with dimethyl polysiloxane. Conventional "hard contact lenses" which cover only the cornea of the eye, usually consist of poly(methyl methacrylate) cross-lined with ethylene glycol dimethyacrylate.

The improved methods for cleaning and disinfecting contact lenses in the same solution in a single step are carried out by heating the lenses in an enzyme-containing solution which is both effective, safe and nontoxic. The single-step method both cleans and disinfects contact lenses in 60 minutes or less.

The cleaning solution may include, in addition to one or more proteolytic enzyme, also carbolytic and lipolytic enzymes, either individually or in combinations. Such enzymes include those derived from plant sources, animal sources and microorganisms, including bacteria and molds. Typical examples of enzymes useful herein include, but are not limited to papain, chymopapain, pancreatin, pepsin, trypsin, chymotrypsin, protease, amylase, lipase, ficin, bromelin, streptokinase, streptodornase, etc. Protease and amylase derived from *Bacillus* and *Streptomyces* bacteria and *Aspergillus* mold have been found to perform well in the single-step cleaning and disinfecting method of the present invention. Enzymatic cleaning solutions prepared principally with microbial protease and amylase and optionally, lipase are described in U.S. Patent 4,690,773 Thus, protease and amylase derived, for example. from a *Bacillus* or *Streptomyces* bacteria or an *Aspergillus* mold effectively remove protein and carbohydrate films resulting from eye secretions and tears without damaging lenses. They are substantially odor-free, non-allergenic, do not require activator/stabilizer, and dissolve completely in aqueous solutions. Such bacterial enzymes are readily available, for example, from the Enzyme Development Corporation, Keyport, New Jersey under the Enzeco Trademark, which includes a food grade of Protease AP I. Other grades of bacterial proteases are commercially available and suitable for use in the methods described herein.

The single-step cleaning and disinfecting process is conducted by immersing the lenses in an aqueous solution of the enzyme cleaner. For purposes of the present invention-aqueous solution-includes water, but preferably solutions adjusted with tonicity agents to approximate the osmotic pressure of normal lacrimal fluids which is equivalent to a 0.9% solution of sodium chloride or 2.5% solution of glycerol are used. The aqueous solutions of enzymes are preferably made isotonic to avoid potential discomfort to the user of the lenses after they are cleaned, disinfected and reinserted.

The aqueous enzyme solutions preferably contain preservatives which are compatible with and do not precipitate in the presence of the enzymes, and comprise concentrations thereby ranging from about 0.00001 to about 0.5 weight percent, and more preferably, from about 0.0001 to about 0.1 weight percent. Suitable preservatives include, but are not limited to thimerosal, sorbic acid, 1,5-pentanedial, alkyl triethanolamines, phenylmercuric salts e.g. nitrate, borate, acetate, chloride and mixtures thereof. Other germicidal compounds and salts may be used, such as salts which are soluble in water at ambient temperature to the extent of at lesat 0.5 weight percent. These salts include the gluconate, isothionate (2 - hydroxyethanesulfonate), formate, acetate, glutamate, succinamate, monodiglycollate dimethanesulfonate, lactate, diisobutyrate and glucoheptonate.

In most instances, the aqueous enzyme cleaning and disinfecting solutions will contain various sequestering or chelating agents to bind metal ions, such as calcium, which might otherwise react with protein and collect on lens surfaces. Ethylenediaminetetraacetic acid (EDTA) and its salts (disodium) are preferred examples. Chelating agents are normally employed in amounts from about 0.1 to about 2.0 weight percent.

In addition to the tonicity agents, preservatives and chelating agents mentioned above the aqueous solutions containing the enzyme(s) may also include buffering agents and surfactants. Suitable buffers include, for example, sodium or potassium citrate, citric acid, boric acid, sodium borate, sodium bicarbonate and various mixed phosphate buffers, including combinations of $Na_2 HPO_4$, $NaH_2PO_4$ and $KH_2PO_4$. Generally, buffers may be used in amounts ranging from about 0.5 to about 2.5%, and more preferably, from about 0.1 to 1.5% by weight.

In some instances, it may be desirable to include in the aqueous enzyme cleaning solutions surface active agents, preferably neutral or non-ionic types for their supplemental cleaning and conditioning properties. Surface active agents may be used generally in amounts ranging up to 15 weight percent. Examples of surfactants suitable for use in conjunction with the enzymes include polyethylene glycol esters of fatty acids e.g. coconut, polysorbate, polyoxyethylene, polyoxypropylene ethers of higher alkanes ($C_{12}$-$C_{18}$). Examples of preferred surfactants include polysorbate 20 (available under the trademark Tween 20), polyoxyethylene (23), lauryl ether (Brij.® 35), polyoxyethylene (40) stearate (Myrj.®) polyoxyethylene (25), propylene glycol stearate (Atlas® 12).

Also included within the group of surfactants noted above is a group of nonionic surface active agents consisting of a poly(oxypropylene) - poly(oxyethylene) adduct of ethylene diamine having a molecular weight from about 7500 to about 27,000 wherein at least 40 weight percent of said adduct is poly(oxyethylene) has been found to be particularly useful in cleaning and conditioning both soft and hard contact lenses in amounts from about 0.01 to about 15 weight percent. Such surfactants are available from BASF-Wyandotte under the trademark-Tetronic.

Lenses are covered with the above aqueous enzyme solutions, usually in a lens case, and are heated to a temperature which will disinfect and allow cleaning in one-step in the same solutions. This is carried out by a cycle consisting of a heating phase and a cooling phase. The heating phase consists of gradually elevating the temperature of the solution from ambient temperature to a maximum temperature of between 60° and 100°C, and preferably between 60° and 85°C, and more preferably between 65° and 75°C. When the maximum temperature has been reached the temperature is maintained for not more than 20 minutes, and more often for about 5 to about 15 minutes.

Although the precise mechanism for the cleaning reaction remains uncertain, the activity of the enzymes for example, in denaturing and removing protein from lens surfaces is believed to be enhanced as the temperature rises. Likewise, when the maximum temperature has been reached during the heating phase and maintained for about 10 minutes, this action operates automatically to inactivate the enzyme terminating the cleaning process while simultaneously disinfecting the lenses. At the conclusion of the heating phase and cooling phase commences whereby the cleaned and disinfected lenses and the inactivated cleaning solution are allowed to cool to ambient temperature. The lenses are then ready for reinserting onto the eyes.

The process is most conveniently conducted with any of the well known commercially available contact lens heat disinfecting units, such as those available from Bausch & Lomb under the trademark Aseptron. Such that disinfecting units, in most instances, can be adapted to the present single-step process. They have temperature profiles which typically include heating up to 80°C which temperature is maintained for approximately 10 minutes, the entire cycle taking about 60 minutes. Temperature profiles of heat units can be modified depending on the type of lens, where for instance, extended wear type contact lenses may be treated to even more abbreviated cleaning and disinfecting cycles and at lower temperature ranges to minimize the potential for both physical damage, discoloration, etc.

The following specific examples demonstrate the method of the subject invention.

Example I

In order to evaluate the performance of the single-step cleaning and disinfecting method and compare it to the conventional two-step method, the following enzyme formulations are prepared:

Water soluble, non-effervescent tablet

Water-soluble, non-effervescent tablest are prepared with each tablet containing about 25 to 30 mg of Protease AP I enzyme commercially available under the Enzeco trademark from Enzyme Development Corporation Keyport, New Jersey. The enzyme is derived from a Bacillus bacteria and contains principally protease and a-amylase activity. The protease activity is approximately 53 casein units/mg. The enzyme is stable at a pH of between 5.0 and 10.0.

The enzyme powder is first granulated with a sufficient amount of a pharmaceutical grade polyethylene glycol (4000) or other suitable binder and lubricant. The granulated fines are then formed into compressed tablets.

Water-soluble effervescent tablet

Effervescent enzyme cleaning tablets are made by preparing an effervescent excipient containing sodium bicarbonate, citric acid and sodium chloride in a weight ratio of 3:1:1. Each of the salts is finely ground separately in a mortar and then mixed tpgether with the aid of a mortar and pestle. A small amount of distilled water e.g.-<0.5 ml is added to the mixture and further blended to initiate molecular interaction of the salts. The mixture is spread evenly on a glass plate and placed in a vacuum oven for 2 to 3 hours at 60°C. The mixture is then finely ground in a mortar and blended with Enzeco Protease AP I enzyme powder in a ratio of excipient to enzyme of 2:1 to provide about 25 to 30 mg of enzyme per tablet. Tablets are then made by compressing at $1.724 \times 10^8$ Pa (2500 psig).

Example II

A clear artificial tear solution is prepared consisting of 0.2 grams of lysozyme/100 ml of electrolyte. The electrolyte is a stock solution prepared from sodium bicarbonate 2.2 gpl, sodium chloride 7 gpl, calcium chloride 0.0005 gpl and potassium chloride 1.5 gpl.

Eight (8) polymacon soft contact lenses commercially available from Bausch & Lomb under the registered trademark Soflens are microscopically inspected before coating with the lysozyme solution. The lenses are then soaked in the lysozyme solution for 30 to 60 minutes at room temperature. The lenses are then placed individually into the wells of Lensgard® carrying cases and placed in Bausch & Lomb Aseptron® heat units in order to denature the lysozyme protein. This procedure is repeated for 10 cycles on the same lenses.

Before initiating the cleaning process, light transmission readings for each of the lenses is measured with a Bausch & Lomb brand Spectronic 2000 Model Ultraviolet/Visible Spectroscope at the visible light range of 500 nm after rubbing the lenses and rinsing with isotonic saline. The lenses are then placed in Bausch & Lomb Lensgard® cases. Each well is filled 2/3 full with isotonic saline solution. A water soluble, non-effervescent tablet from Example I is placed in seven (7) of the lens wells and an effervescent tablet from Example I is placed in the remaining lens well. The cases are closed and shaken to insure dissolution of the tablets. Cases 1-5 containing non-effervescent tablets are placed in Aseptron heat disinfecting units having about a 60 minute cycle with a maximum heating temperature of about 80°C. At the end of the cycle, the lenses are removed, rubbed and rinsed in isotonic saline. Cases 6 and 7 also containing cleaning solution made from non-effervescent tablets and case 8 containing solution from the effervescent tablet are allowed to soak overnight at room temperature. Cases 6-8 are then emptied, filled with distilled water and then placed in Aseptron units for one cycle to disinfect the lenses. At the completion of the cycle the lenses are removed, rubbed and rinsed in isotonic saline. Post cleaning light transmission readings are taken on all the lenses.

TABLE

| Lens | One step | Two step | Tablet | Pre-clean % transmission | Post-clean % transmission |
|------|----------|----------|--------|--------------------------|---------------------------|
| 1 | X | | Non-effervescent | 90.5 | 95.6 |
| 2 | X | | " | 90.3 | 95.1 |
| 3 | X | | " | 80.7 | 96.1 |
| 4 | X | | " | 92.8 | 94.8 |
| 5 | X | | " | 84.0 | 95.1 |
| 6 | | X | " | 84.4 | 94.1 |
| 7 | | X | " | 76.1 | 96.2 |
| 8 | | X | Effervescent | 85.7 | 97.3 |

A lens post clean light transmission of 94 percent or more is considered cosmetically clean. Accordingly, the data in the table demonstrates the single-step cleaning and disinfecting method performs favorably against the conventional two step method.

**Claims**

1. A method of (1) cleaning contact lenses of proteinaceous and other deposits using a proteolytic enzyme in aqueous solution, and (2) disinfecting the contact lenses, characterized in that it is a single step method providing simultaneous cleaning and disinfection which comprises placing the lenses in a solution comprising the proteolytic enzyme dissolved in water and then subjecting the solution and lenses to a heating cycle consisting of a heating phase followed by a cooling phase, said heating phase consisting of gradually elevating the temperature of the solution from ambient temperature to a maximum temperature between 60° and 100°C and then maintaining said maximum temperature for not more than 20 minutes, the total time for which the lenses are heated at between 60°C and 100°C being 60 minutes or less, sufficient to clean and disinfect the lenses and inactivate the proteolytic enzyme.

2. A method according to Claim 1, wherein the period of time for which the solution and lenses are heated to an elevated temperature is between 5 minutes and 15 minutes.

3. A method according to any preceding claim, wherein the enzyme solution contains papain.

4. A method according to any preceding claim, wherein the enzyme solution contains pancreatin.

5. A method according to any preceding claim, wherein the contact lenses are soft contact lenses.

6. A method according to any preceding claim, wherein the protease-containing solution includes amylase.

7. A method according to Claim 6, wherein the amylase is a microbial amylase.

8. A method according to any one of Claims 6 or 7, wherein the protease is derived from a *Bacillus* or *Streptomyces* bacteria or an *Aspergillus* mold.

9. A method according to any one of Claims 6-8, wherein the elevated temperature is between 60°C and 85°C.

10. A method according to Claim 9, wherein the elevated temperature is between 65°C and 75°C.

11. A method according to any one of Claims 6-10, wherein the contact lenses are of the type having a water content of at least 55% by weight.

12. A method according to any one of Claims 6-11, wherein the solution is substantially isotonic with lacrimal fluids.

**Patentansprüche**

1. Verfahren zur (1) Reinigung von Kontaktlinsen von proteinartigen und anderen Ablagerungen unter Verwendung eines proteolytischen Enzyms in wäßriger Lösung und (2) zur Desinfektion der Kontaktlinsen, **dadurch gekennzeichnet,** daß es ein gleichzeitig reinigendes und desinfizierendes Einstufenverfahren ist, bei dem die Linsen in eine Lösung gelegt werden, die proteolytisches Enzym gelöst in Wasser enthält, und danach die Lösung und die Linsen einem aus einer Erwärmungsphase gefolgt von einer Abkühlungsphase bestehendem Erwärmungszyklus unterworfen werden, wobei die Erwärmungsphase aus dem allmählichen Erhöhen der Temperatur der Lösung von Raumtemperatur auf eine Maximaltemperatur zwischen 60 und 100°C und anschließendem Aufrechterhalten der Maximaltemperatur für nicht länger als 20 Minuten besteht und wobei die Gesamtzeit, die die Linsen auf zwischen 60 und 100°C erwärmt werden, 60 Minuten oder weniger beträgt und ausreicht, um die Linsen zu reinigen und zu desinfizieren und das proteolytische Enzym zu inaktivieren.

2. Verfahren nach Anspruch 1, bei dem die Dauer der Erwärmung der Lösung und der Linsen auf eine erhöhte Temperatur zwischen 5 Minuten und 15 Minuten liegt.

3. Verfahren nach einem der vorangehenden Ansprüche, bei dem die Enzymlösung Papain enthält.

4. Verfahren nach einem der vorangehenden Ansprüche, bei dem die Enzymlösung Pancreatin enthält.

5. Verfahren nach einem der vorangehenden Ansprüche, bei dem die Kontaktlinsen weiche Kontaktlinsen sind.

6. Verfahren nach einem der vorangehenden Ansprüche, bei dem die Protease enthaltende Lösung Amylase enthält.

7. Verfahren nach Anspruch 6, bei dem die Amylase eine mikrobische Amylase ist.

8. Verfahren nach einem der Ansprüche 6 - 7, bei dem die Protease aus Bacillus- oder Streptomyces-Bakterien oder einem Aspergillus-Schimmelpilz stammt.

9. Verfahren nach einem der Ansprüche 6 - 8, bei dem die erhöhte Temperatur zwischen 60 und 85°C liegt.

10. Verfahren nach Anspruch 9, bei dem die erhöhte Temperatur zwischen 65 und 75°C liegt.

11. Verfahren nach einem der Ansprüche 6 - 10, bei dem die Kontaktlinsen von dem Typ mit einem Wassergehalt von zumindest 55 Gew.% sind.

12. Verfahren nach einem der Ansprüche 6 - 11, bei dem die Lösung gegenüber Tränenflüssigkeiten im wesentlichen isotonisch ist.

## Revendications

1. Procédé pour (1) nettoyer des lentilles de contact en en retirant des dépôts protéiques et autres au moyen d'une enzyme protéolytique en solution aqueuse, et (2) désinfecter les lentilles de contact, caractérisé en ce que c'est un procédé en un seul stade assurant simultanément le nettoyage et la désinfection, qui consiste à placer les lentilles dans une solution comprenant l'enzyme protéolytique dissoute dans l'eau, puis à soumettre la solution et les lentilles à un cycle de chauffage consistant en une phase de chauffage suivie d'une phase de refroidissement, ladite phase de chauffage consistant en une élévation graduelle de la température de la solution de la température ambiante à une température maximum comprise entre 60°C et 100°C puis à un maintien de cette température maximale pendant une durée ne dépassant pas 20 minutes, la durée totale durant laquelle les lentilles sont chauffées entre 60°C et 100°C étant de 60 minutes ou moins, suffisante pour nettoyer et désinfecter les lentilles et inactiver l'enzyme protéolytique.

2. Procédé selon la revendication 1 dans lequel la durée pendant laquelle on chauffe la solution et les lentilles à une température élevée est comprise entre 5 minutes et 15 minutes.

3. Procédé selon l'une quelconque des revendications précédentes, dans lequel la solution enzymatique contient de la papaïne.

4. Procédé selon l'une quelconque des revendications précédentes, dans lequel la solution enzymatique contient de la pancréatine.

5. Procédé selon l'une quelconque des revendications précédentes, dans lequel les lentilles de contact sont des lentilles de contact souples.

6. Procédé selon l'une quelconque des revendications précédentes dans lequel la solution renfermant la protéase contient une amylase.

7. Procédé selon la revendication 6, dans lequel l'amylase est une amylase microbienne.

8. Procédé selon l'une des revendications 6 ou 7, dans lequel la protéase provient d'une bactérie du genre Bacillus ou Streptomyces ou d'une moisissure du genre Aspergillus.

9. Procédé selon l'une quelconque des revendications 6 à 8, dans lequel la température élevée est comprise entre 60°C et 85°C.

10. Procédé selon la revendication 9, dans lequel la température élevée est comprise entre 65°C et 75°C.

11. Procédé selon l'une quelconque des revendications 6 à 10, dans lequel les lentilles de contact sont de type ayant une teneur en eau d'au moins 55% en poids.

12. Procédé selon l'une quelconque des revendications 6 à 11, dans lequel la solution est pratiquement isotonique par rapport aux fluides lacrimaux.